Europäisches Patentamt

European Patent Office

Office européen des brevets

(11) Publication number: **0 465 436 A1**

# EUROPEAN PATENT APPLICATION

(21) Application number: **91830219.1**

(22) Date of filing: **23.05.91**

(51) Int. Cl.⁵: **A61F 2/36,** A61F 2/30, A61B 17/16

(30) Priority: **22.06.90 IT 2072190**

(43) Date of publication of application: **08.01.92 Bulletin 92/02**

(84) Designated Contracting States: **BE CH DE ES FR GB LI NL SE**

(71) Applicant: **G. CREMASCOLI S.R.L. Via Clemente Prudenzio, 14/16 I-20138 Milano (IT)**

(72) Inventor: **Ghisellini, Franco, c/o G. CREMASCOLI S.r.l Via Clemente Prudenzio, 14/16 I-20138 Milano (IT)**

(74) Representative: **Cicogna, Franco Ufficio Internazionale Brevetti Dott.Prof. Franco Cicogna Via Visconti di Modrone, 14/A I-20122 Milano (IT)**

(54) **Replacement hip prosthesis stem adapted to be locked at several locking positions.**

(57) The present invention relates to a replacement hip prosthesis stem (1) adapted to be locked at several locking positions, comprising an elongated body including, at the top end portion thereof and at its proximal portion, an enlarged portion ending with a neck (3) at the apex of which there is arranged a coupling cone element (4) for the prosthesis small heads, and, at the bottom or distal end portion thereof, being provided with a constant cross-section stem (10).

On the enlarged portion top holes (20) are formed which have their axes perpendicular to the axes of bottom holes (25) formed through the constant cross-section stem (10), the bottom (25) and top (20) holes being adapted to receive cross locking screws.

EP 0 465 436 A1

Fig. 1

## BACKGROUND OF THE INVENTION

The present invention relates to a replacement hip prosthesis stem adapted to be locked at several locking positions.

As is known, during replacement or new operations on a hip prosthesis, that is as a femoral stem must be removed because of possible complications, in order to replace this stem with another new stem, there is conventional used a prosthesis stem which is usually called "replacement stem" which has been specifically designed for such an operation.

Prior replacement hip stems are usually provided with a plurality of holes extending through the stem top or proximal end portion, these holes being provided for receiving cross locking screws.

However, sometimes these locking screws do not provide a sufficient locking force, mainly because these screws affect only a very reduced region of the prosthesis stem and, moreover, because they are conventionally applied on a bone region previously affected by the original stem, and which, accordingly, can not firmly support the replacement or new stem.

Another drawback is that these prior replacement prosthesis stems do not provide for an easy application and a proper locating of the locking cross screws.

## SUMMARY OF THE INVENTION

Accordingly, the aim of the present invention is to overcome the above mentioned drawbacks, by providing a replacement hip prosthesis stem adapted to be locked at several locking positions, which, in particular, can be firmly locked with respect to the femoral bone at several regions, and, expecially, at regions of the femoral bone which have not been affected by the original prosthesis stem removed from the bone.

Within the scope of the above mentioned aim, a main object of the present invention is to provide such a replacement hip prosthesis stem which is adapted to properly discharge the applied stresses, thereby providing a very firm locking mainly with respect to rotary movements on a cross plane, that is twisting movements.

Another object of the present invention is to provide such a prosthesis stem in which the locking screws can be quickly applied and centered as the stem is engaged within the medullar and femoral hollow.

Yet another object of the present invention is to provide such a replacement hip prosthesis stem which can be easily made by using conventional making methods and which, moreover, is very reliable and safe in operation.

According to one aspect of the present invention, the above mentioned aim and objects, as well as yet other objects, which will become more apparent thereinafter, are achieved by a replacement hip prosthesis stem adapted to be locked at several locking positions, characterized in that said stem comprises an elongated stem body provided, at its top or proximal end portion, with an enlarged portion ending with a neck, at the apex of which there is arranged a cone element for coupling the prosthesis heads and, at the bottom or distal end thereof being provided with a constant cross-section stem, through said enlarged portion there being formed top holes the axes of which are perpendicular to the axes of bottom holes formed through said constant cross-section stem, said bottom and top holes being adapted to receive cross locking screws.

## BRIEF DESCRIPTION OF THE DRAWINGS

Further characteristics and advantages of the present invention will become more apparent from the following disclosure of a preferred, though not exclusive, embodiment thereof, which is illustrated, by way of an indicative, but not limitative, example, in the figures of the accompanying drawings, where:

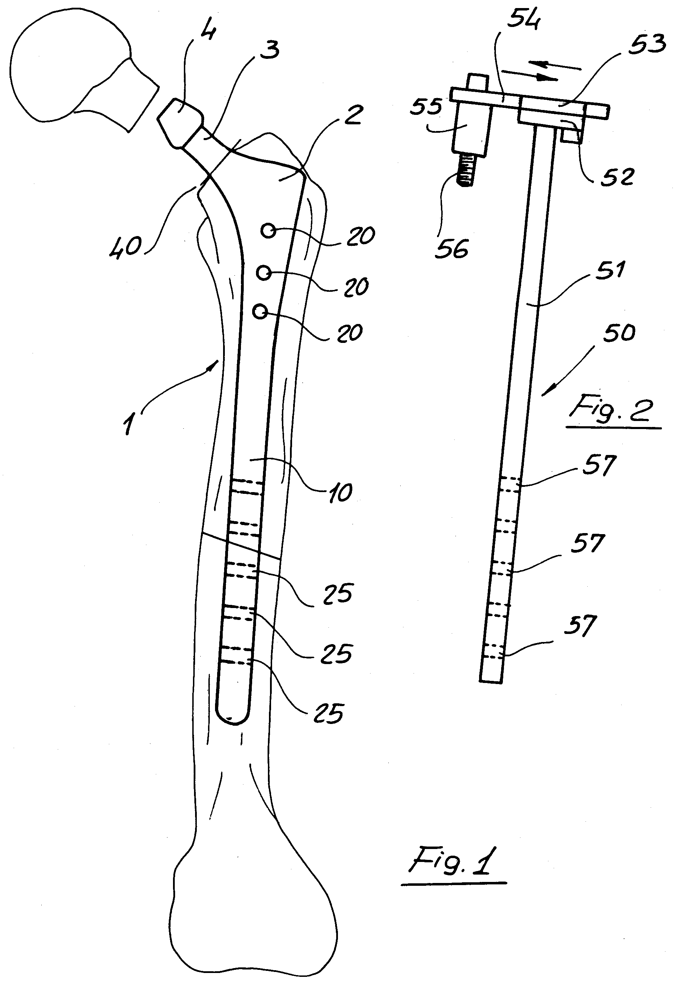

Figure 1 shows the hip prosthesis stem according to the invention as engaged in a femoral bone.

Figure 2 is a schematic view illustrating an apparatus for applying locking screws to the prosthesis stem, as engaged in the femoral medullar hollow.

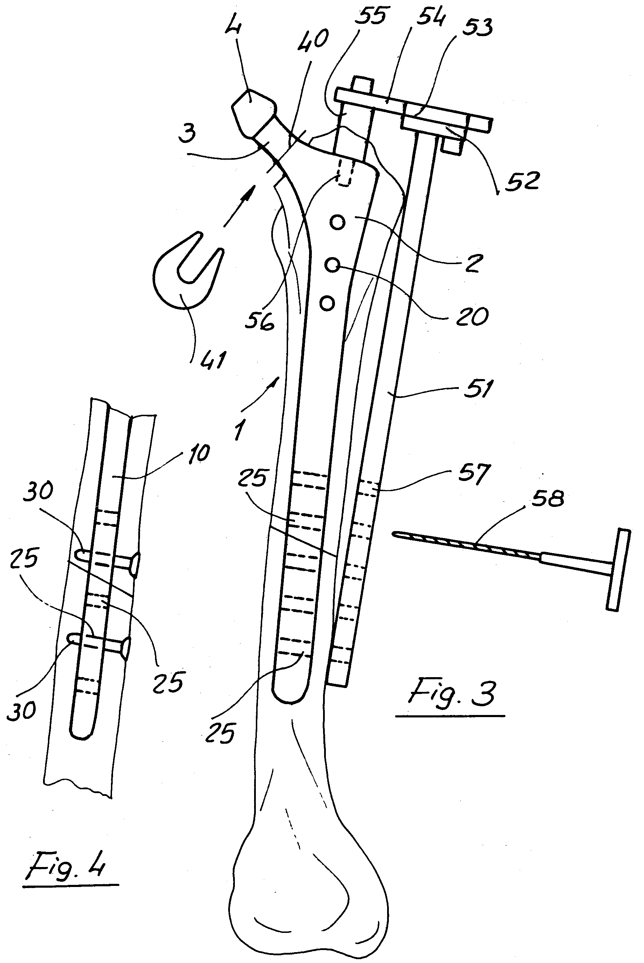

Figure 3 illustrates the prosthesis stem engaged in the bone and cooperating with an apparatus for making holes through the femoral bone.
and

Figure 4 shows a method for introducing the locking screws.

## DESCRIPTION OF THE PREFERRED EMBODIMENT

With reference to the figures of the accompanying drawings, the replacement hip prosthesis stem, adapted to be locked at several locking positions according to the invention, which has been overally indicated at the reference number 1, is provided with an elongated stem body which can be introduced into the bottom of the femoral medullar hollow so as to arrive at very strong bone regions, to be firmly locked therein.

The stem 1 is provided, at its top or proximal end, with an enlarged portion 2, ending at a neck 3, at the apex of which there is provided a cone element 4, for coupling the conventional prosthesis heads.

Near its bottom end, the enlarged portion is coupled to a constant cross-section stem 10, which has a constant diameter substantially equal to the thickness of the enlarged portion.

Through the enlarged portion, in particular, there are formed three or more holes 20, which are adapted to receive throughgoing locking screws, suitable to lock at a desired position the prosthesis stem with respect to the femoral bone.

Through the distal or bottom portion, the length of which corresponds to about 2/3 of the overall length of the femoral stem, there are provided bottom holes 25, the axes of which are mutually parallel and perpendicular to the axes of the top holes 20.

The provision of the top holes and bottom holes with a mutual offset of 90°, allows the locking screws, indicated at 30, and which are turned by 90° between the bottom location and top location, to be engaged in the femoral bone without excessively weakening the stem at the upermost portion thereof.

The axes of the holes, at the distal region, are so arranged that the system can be easily located: in fact, the presence of vessels, nervous fibres and tissues would make inpractical a different insertion direction.

At the top portion of the stem, and more specifically at the cutting plane thereof, there are provided slots 40, slanted at 45°, which are adapted to receive a bearing collar 41 for allowing the prosthesis to bear on the cutting plane.

This requirement is due to contingent operation conditions, such as the femoral bone condition, the width of the endomedullar spaces, the shape of the femoral channel and the like.

In this connection it should be apparent that the possibility of using a variable number of screws, engaged in the top holes and bottom holes, is particularly important in the presence of fractures of the femoral diaphysis.

In fact, under such a condition, the stem will operate as an endomedullar osteosynthesis nail, to lock to one another the two lengths so as to stabilize the facing edges of the fracture.

It is to be pointed out that the position and number of the locking screws will depend on the type and shape of the fracture: under some particular cases, the screws can also be omitted.

In order to properly locate the screws, a screw application apparatus is provided, indicated overally at the reference number 50, which substantially comprises an outer rod element 51, coupled at the top thereof to a block 52 having a coupling slide 53 for a cross element 54 provided for coupling to an end piece 55 including a coupling screws 56 which, in turn, can be engaged in a threaded hole formed on the top portion of the stem already engaged in the medullar channel or hollow.

This rod, as locked in a sqaure seat, is parallel to the prosthesis stem and is provided with registering holes 57 which are aligned with the corresponding stem holes, so as to operate as guiding elements, in order to form the holes for the screws 30, by means of a drill, generally indicated at the reference number 58.

The double-slide block 53 allow the rod to be moved toward the bone as far as possible, so as to form in a very accurate way the holes for the screws 30.

From the above disclosure it should be apparent that the invention fully achieves the intended aim and objects

In particular, it should be pointed out that a replacement hip prosthesis stem has been provided which can be firmly locked in the femoral bone.

While the invention has been disclosed and illustrated with reference to a preferred embodiment thereof, it should be apparent that the disclosed embodiment is susceptible to several modifications and variations, all of which will come within the spirit and scope of the appended claims.

## Claims

1. A replacement hip prosthesis stem adapted to be locked at several locking positions, characterized in that said stem comprises an elongated stem body provided, at its top or proximal end portion, with an enlarged portion ending with a neck, at the apex of which there is arranged a cone element for coupling the prosthesis heads and, at the bottom or distal end thereof being provided with a constant cross-section stem, throgh said enlarged portion there being formed top holes the axes of which are perpendicular to the axes of bottom holes formed through said constant cross-section stem, said bottom and top holes being adapted to receive cross locking screws.

2. A replacement stem, according to Claim 1, characterized in that said constant cross-section stem has a diameter which is substantially equal to the thickness of the enlarged portion.

3. A replacement stem, according to Claims 1 and 2, characterized in that said stem comprises, on said neck, at the cutting plane thereof, a plurality of slots slanted at 45° with respect to the longi tudinal axis of the stem, said slots being adapted to removably receive bearing collar elements.

4. A replacement stem, according to one or more of the preceding claims, characterized in that said constant cross-section stem substantially extends for 2/3 of the longitudinal extension of said prosthesis stem.

5. An apparatus for applying locking screws to a prosthesis replacement stem according to one or more of the preceding claims, characterized in that said apparatus comprises a rod element including, at its top end, a block coupled, through a cross element, to an end piece having a coupling screw for engaging in a threaded hole formed at the proximal end portion of said prosthesis stem, said rod being provided with registering holes for facilitating the drilling of the bone at the bottom holes of said prosthesis stem.

6. An apparatus, according to Claim 5, characterized in that said block is coupled to said cross element with the interposition of a slide element adapted to allow said rod to be displaced toward said prosthesis stem engaged in the endomedullar hollow of the femoral bone.

Fig. 2

Fig. 1

Fig. 3

Fig. 4

**European Patent Office**

**EUROPEAN SEARCH REPORT**

Application Number

EP 91 83 0219

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 895 572 (I. CHERNOFF)<br>* Column 3, line 64 - column 4, line 23; figures *<br>--- | 1,2,4 | A 61 F 2/36<br>A 61 F 2/30<br>A 61 B 17/16 |
| Y | US-A-3 814 089 (W.M. DEYERLE)<br>* Figures 3,4 * | 1,2,4 | |
| X | | 5 | |
| | --- | | |
| A | EP-A-0 095 440 (MECRON)<br>* Page 8, line 31 - page 9, line 8; page 10, lines 19-26 *<br>--- | 1,2,4 | |
| X | DE-U-8 906 537 (W. ROTH)<br>* Page 4, lines 18-21; page 6, lines 18-27; figures 1,2 * | 5,6 | |
| A | | 1 | |
| | --- | | |
| A | US-A-4 770 660 (R.G. AVERILL)<br>* Column 3, lines 13-23; figures *<br>--- | 3 | |
| A | EP-A-0 158 014 (SULZER)<br>* The abstract; figures 4,5 *<br>--- | 3 | TECHNICAL FIELDS SEARCHED (Int. Cl.5) |
| A | FR-A-2 501 998 (J. BABIN-CHEVAYE)<br>* Page 3, lines 11-24; page 3, line 35 - page 4, line 13; figures *<br>--- | 1-4 | A 61 F<br>A 61 B |
| A | DE-A-3 205 577 (HOWMEDICA)<br>* Page 34, lines 6-20; page 35, lines 6-10; figures 13-15,31-33 *<br>----- | 1 | |

The present search report has been drawn up for all claims

| Place of search | Date of completion of the search | Examiner |
|---|---|---|
| THE HAGUE | 19-09-1991 | WOLF C.H.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)